# EUROPEAN PATENT APPLICATION

(11) **EP 4 495 228 A1**
(43) Date of publication of application: **22.01.2025**
(21) Application number: 23186533.8
(22) Date of filing: 19.07.2023
(51) Int. Cl.: C12N 5/071, A61K 35/12

(54) **METHOD FOR PRODUCING EXTRACELLULAR VESICLES**

(71) Applicant: ConvEyXo, 6041 Charleroi (BE)
(72) Inventor: PRIEELS, Jean Paul, 1380 Lasne (BE); SMAL, Jean, 1030 Schaerbeek (BE); SONNAERT, Maarten, 3012 Leuven (BE); SCHROOTEN, Jan, 3000 Leuven (BE); HUBAUX, Roland, 5030 Gembloux (BE)
(74) Representative: Calysta NV

(57) **Abstract**

Method for the production of extracellular vesicles comprising a supply of a bioreactor containing a porous three-dimensional scaffold having a specific surface area and a total volume by a culture medium inside the bioreactor, a seeding the scaffold having a total volume of scaffold with adherent cells resuspended after detachment, a phase of attachment of said adherent cells resuspended after detachment on the scaffold, an expansion of the adherent cells on the scaffold, a production of extracellular vesicles by the adherent cells during a production phase, and a harvest of extracellular vesicles, wherein the porous 3D scaffold is a porous 3D printed scaffold, having a ratio between said specific surface area and said total volume comprised between 25 and 250, on which said adherent cells expanded are immortalized cells from an immortalized cell line, said immortalized cells having a multiplication factor of at least 5 during the expansion during a single expansion step in said scaffold.

## Description

### Technical Field

The present invention relates to a method for producing extracellular vesicles compatible with the pharmaceutical industry, for example exosomes, using a culture of adherent cells on a porous three-dimensional scaffold.

### Technological background of the invention

Exosomes are extracellular vesicles with a diameter between 30 and 150 nm and are secreted by a multitude of cell types. Exosomes are involved in several cellular processes such as intercellular communication, antigen presentation, transfer of proteins, messenger RNAs, siRNAs, and/or microRNAs from one cell to another. The function of exosomes is altered in many diseases such as cancers, which suggests their importance as a diagnostic tool and/or in therapeutic applications, for example in acellular therapies as a vehicle to deliver therapeutic molecules, in immunotherapy, to treat different types of cancer and/or inflammatory and cardiovascular neurodegenerative diseases, diabetes, etc.

Some protocols for isolating and purifying exosomes and extracellular vesicles are known, and exosomes have already been successfully used in cell-free therapy studies.

However, although some exosome isolation and purification protocols exist, it is important to develop exosome production and purification methods that allow for batch-to-batch consistent pharmaceutical-grade exosome production at large scale, with high yield, while being fast and inexpensive so as to increase the use of exosomes as a cell-free therapy tool in medical practice, for the benefit of the greatest number of patients.

### State of the art

The production of exosomes must meet several constraints: it is necessary to obtain a culture of cells producing high density exosomes of high quality, such as for example mesenchymal stem cells, and to put these cells under adequate conditions to maximize their production of exosomes, in such a way as to obtain a quantity of exosomes sufficient to be able to be used in therapeutic applications. Extracellular vesicles from mesenchymal stem cells have been shown to offer broad regenerative potential in many diseases.

There are methods for producing exosomes using adherent cell culture on a porous three-dimensional scaffold. The porous three-dimensional scaffold being known to ensure a high cell density of cells in culture.

From document EP3990625 a method for producing extracellular vesicles from stem cells using a perfused bioreactor is known. The stem cells according to this document can be adult stem cells, embryonic stem cells, induced pluripotent stem cells, umbilical cord blood stem cells or amniotic fluid stem cells. The examples of EP3990625 mention human dental pulp stem cells (DPSC) at passages 7-9 and adipose-derived mesenchymal stem cells. This document describes a method for improving the production and/or the secretion of extracellular vesicles from at least one porous three-dimensional scaffold having a population of stem cells adherent to this scaffold. According to this document, the porous three-dimensional scaffold comprises at least one material chosen from the group consisting of: polyester, polypropylene, polylactic acid (PLA), poly-L-lactic acid (PLLA), poly(lactic-co-glycolic acid) (PLGA), polycaprolactone (PCL), cellulose, silk, glass and natural and synthetic hydrogels selected from: gelatin, collagen, fibrin, PEG, alginate and chitosan. Moreover, the method according to this document uses various conditions of shear stresses on a variety of stem cells to increase the secretion of extracellular vesicles. In this document, the shear stresses can come from a displacement of the porous three-dimensional scaffold inside the flow chamber. This displacement can come from an agitation, vibration, rotation, undulation or inclination of the porous three-dimensional scaffold. A complex device is therefore necessary to ensure the displacement of the porous three-dimensional scaffold. In addition, the movement of the porous three-dimensional scaffold can create a lot of mechanical stress on the support itself, which can cause, among other things, premature wear of the support.

It is also known from document WO202238598 a method for producing extracellular vesicles from muscle cells. The method according to this document comprises a step according to which a multi-layered three-dimensional scaffold is generated where elastic microfibers are present in the layers. The method also comprises the seeding of the scaffold by a population of muscle cells and a culture of this population of muscle cells on this scaffold as well as the secretion of extracellular vesicles in the culture medium. More particularly, according to this document, the three-dimensional scaffold (polydimethylsiloxane polymers, PDMS) is prepared using a silicone elastomer base and a silicone elastomer curing agent. The scaffold is seeded by primary human skeletal muscle cells (SkMCs) in a skeletal muscle cell growth medium. After 3 weeks of culture, the cells forming multi-layered muscle fibres are subjected to stretching (cyclic tension) for 2 days in order to produce exosomes. Unfortunately, primary cells having a limited lifespan. As a consequence, the method described in this document is not suitable for the development and the optimisation of the extracellular vesicles production process as well as for the extracellular vesicle production at large scale. In addition, the method is not easily automatable for repeated and reproducible productions of exosomes necessary for current medical practice and this method is also not easily transferable, or even not relevant, to other cell types.

It is also known from document CN109628392 a method for the production of large-scale exosomes of clinical quality that meets the regulations of good production/manufacturing practices (GMP). According to this document, a cell pellet is obtained from a patient sample (typically an effusion from the patient's knee joint). Then, the mesenchymal stem cells from the cell pellet are inoculated, by injecting these cells onto microcarriers for three-dimensional cell culture. The cell culture uses a serum-free medium. The production and excretion of exosomes in the culture medium is ensured during the culture of the mesenchymal stem cells on the microcarriers. Unfortunately, the use of microcarriers makes the exosome extraction step quite complex using a separation membrane. On the other hand, the culture of primary cells (from a patient sample) also makes the method not very reproducible and automatable, preventing the use of this production method in current medical practice.

Although methods for producing extracellular vesicles already exist on the market, in practice, there is currently no method for producing extracellular vesicles, exosomes, which is both sufficiently reliable, sufficiently generalizable and reproducible while remaining affordable in terms of production costs so that exosomes can be used as a therapeutic means in daily medical practice.

Consequently, there is a need to improve the current production methods by making them more efficient, industrially applicable both in terms of the quantity of exosomes produced and in terms of production costs, and this with a production of exosomes with constant pharmaceutical quality regardless of the production batch.

### Objectives of the invention

The present invention aims to overcome the drawbacks of the state of the art, in particular those described above.

In particular, the present invention proposes to provide a method for producing exosomes making it possible to reach an industrial scale of production which is profitable by minimizing production costs.

Another objective of the present invention is to provide a method of producing extracellular vesicles of constant pharmaceutical quality, regardless of the production batch, which meets GMP standards.

### Summary of the invention:

To achieve the aforementioned objectives, the present invention provides a method for the production extracellular vesicles compatible with the pharmaceutical industry, for example exosomes comprising the following steps:
- the supply of a bioreactor chamber containing a porous three-dimensional scaffold by a cell culture medium via a supply port, and, after a predefined duration, an exit via an outlet port, wherein the porous three-dimensional scaffold having a specific surface area and a total volume,
- seeding said porous three-dimensional scaffold with a starting number of adherent cells resuspended after detachment by feeding said starting number of adherent cells resuspended after detachment in a volume of culture medium,
- a phase of attachment of said adherent cells resuspended after detachment on said three-dimensional scaffold to form adherent cells on said porous three-dimensional scaffold,
- an expansion of the adherent cells on said porous three-dimensional scaffold up to a second number of adherent cells, said second number of adherent cells divided by said starting number defining a multiplication factor,
- a production of extracellular vesicles by said adherent cells during a production phase, subsequent to or concomitantly with the expansion, during which the vesicles are produced and excreted in the culture medium to give a culture medium enriched with extracellular vesicles,
- a harvest of extracellular vesicles,
characterized in that
said porous three-dimensional scaffold is a porous three-dimensional printed scaffold having a ratio between said specific surface area and said total volume of the scaffold comprised between 25 and 250, on which said adherent cells expanded are immortalized cells from an immortalized cell line, said immortalized cells having the multiplication factor of at least 5 for example during a single expansion step in said scaffold.

As it can be seen, according to the present invention, a bioreactor with a porous three-dimensional printed scaffold (fixed bed) is seeded with adherent immortalized cell from an immortalized cell line. This porous three-dimensional printed scaffold (fixed bed), by contrast to chemically obtained 3D scaffold and/or by contrast to 2D scaffold stacked or stuffed to make 3D scaffold, allows to control the scaffold geometry in such a way that there is no preferred fluid pathway inside the scaffold. The porous three-dimensional scaffold with said controlled geometry as a result of the 3D printing method of producing the scaffold has a specific surface area and a total volume. The controlled geometry of the scaffold in 3 dimensions, instead of random geometries of stacked 2D geometries, enables a 3 dimensional fluid flow, thus avoiding preferential pathways. The ratio between the specific surface area and the total volume of the scaffold is comprised, according to the invention, between 25 and 250. The scaffold 3D optimized geometry and the 3D printing method offer an environment where the cells can grow in each direction giving rise to an homogeneous cell distribution, in an uniform way regardless of the position of the cells within the scaffold due to the absence of preferential pathway (homogeneous flow path) while remaining in close contact with their secreted metabolites promoting the growth of the cells.

According to the article from Tang Y. et al. (2021) Advances in mesenchymal stem cell exosomes: a review. Stem Cell Research & Therapy 12, 71, mesenchymal stem cells (MSCs) are used as a source of exosomes production in regenerative medicine where the exosomes produced by MSCs have the advantage to show low immunogenicity and high repairability. In addition, according to the article from Kronstadt SM et al. (2023) Mesenchymal stem cell culture within perfusion bioreactors incorporating 3D-printed scaffolds enables improved extracellular vesicle yield with preserved bioactivity. Adv. Healthcare Mater. 2300584, shows improved EV production in a perfused bioreactor having a 3D-printed scaffold. However, clinical application is challenging as a consequence of cell senescence during *in vitro* expansion, resulting in a low level of exosomes production or resulting in a level of exosomes production that is not maintained for a long time, thus preventing to reach an industrial scale of production and cost-effective clinical applications.

The unique combination of using adherent immortalized cells from an immortalized cell line, which by nature do not encounter senescence and can be cultured over 50 cell generations, with an optimized culture environment such as the porous 3D printed scaffold according to the present invention, allows reaching a uniform but also a reproducible cell growth with a very high yield of cell of high quality. Indeed, according to the present invention, cell densities up to 100000000 (1 E8) cells/cm³ have been obtained.

After or concomitantly to the expansion (growth phase), extracellular vesicles production phase is started, for example by stress or chemical induction, and the vesicles are produced and excreted in the culture medium to give a culture medium enriched with extracellular vesicles. After the production phase, the extracellular vesicles can be harvested, by collecting the culture medium, which latter contains most of the extracellular vesicles, but also contaminating cells. Indeed, upon harvesting the culture medium containing the extracellular vesicles, some detached cells can be collected together representing a contaminant for the extracellular vesicles fraction. When the number of cells is too high, the further purification is complexified.

Surprisingly, while the density of cells after the growing phase is very high, it was found that high extracellular vesicle (EV) concentration is achieved with EV of high quality in the presence of few death cells in the harvesting solution of the extracellular vesicles. According to the present invention, the 3D printed scaffold with a controlled geometry having a specific surface area to volume ratio comprised between 25 and 250 offers not only an adequate environment to promote but also to maintain an uniform cell culture. By the unique combination of this adequate environment with a culture of an immortalized cell line, which are very stable between each generation, and with a multiplication factor of at least 5 allows not only to reach but also to maintain a very high yield of cells of high and constant quality with few death cells. This cell culture of high yield and constant quality regardless of the production batch promotes a production of extracellular vesicles of high and constant pharmaceutical quality, which meets GMP standards while reaching an industrial scale of production which is profitable by minimizing production costs of a cycle of production.

According to the present invention, the term "extracellular vesicle" (EV) is understood to mean a lipid bilayer-delimited particles that can be secreted by numerous cell types. The size of the extracellular vesicles ranges from 20 nm to more than 1000 nm, wherein exosomes represent extracellular vesicles with a size range between 30 and 150 nm, while microvesicles may have a size ranging from 100 nm to 1000 nm and apoptotic bodies have a size greater than 1000 nm.

According to the present invention, the source cell line of EV's is an immortalized cell line, such as immortalized cell line from stem cells, from mesenchymal stem cells (MSC), amniotic stem cells, or induced pluripotent stem (iPS) cells, dendritic cells, hematopoietic cells, epithelial cells, endothelial cells, nerve cells, blood cells or immune cells. Preferably, the source cell line of EVs can be an immortalized cell line from amnion-derived multipotent progenitor cells, chorion derived mesenchymal stem cells, induced pluripotent stem cells, keratinocytes, fibroblasts, embryonic stem cells, ectodermal stromal cells, endodermal stromal cells, mesodermal stromal cells, dental pulp stem cells, non-tumoral or tumoral immortalized cells, immune regulatory cells, or other immortalized cells. Preferably, according to the present invention, the adherent cells are tumoral or non-tumoral immortalized cells, wherein preferably the tumoral or non-tumoral immortalized cells forming part of a group of cells comprising immortalized immune regulatory cells, immortalized mesenchymal stem cells, immortalized Wharton's Jelly derived-mesenchymal stem cells, immortalized bone marrow-derived mesenchymal stromal cells, immortalized hTERT-mesenchymal stem cells, and/or immortalized induced pluripotent stem cells or cells derived from induced pluripotent stem cells. Indeed, immortalized cells as described is very stable with a very large or illimited capacity of proliferation. According to the present invention, preferably, the term " immortalized cells as described is very stable with a very large capacity of proliferation" is understood to be cells able to have a generation number superior to 50, preferably superior to 70, more preferably superior to 100.

According to the present invention, the term "stress or chemical induction" that may allow the starting of the extracellular vesicles production phase comprise (i) a modulation of the culture conditions such as for example modifying the temperature of culture, modifying the pH of the culture medium, modifying the level of 02, (ii) a modification of the biological conditions through modifying the amount of serum and/or cytokines and/or other growth factors and/or other cell culture medium supplements and/or other components of the cell culture media formulation (iii) a chemical stimulation such as for example the addition of sulfhydryl blocking agents, agents for calcium influx, H₂O₂, and/or vesiculation compound, and/or specific inhibitors or activators of cell signalling, or any compound involved in vesicle production, trafficking, and excretion, (iv) physical stimulation such as for example shearing stress, high-frequency ultrasound, irradiation, and/or (v) cell membrane disruption.

According to the present invention, preferably, the scaffold is designed through a computer-aided design, CAD. Preferably, the volumes and/or the specific surface area of the scaffold are determined by high resolution X-ray computed tomography.

According to the present invention, preferably, the term "scaffold" is understood to mean a fixed bed.

Preferably, the bioreactor chamber is included in a bioreactor (bioreactor system) comprising, in addition of the bioreactor chamber containing a porous three-dimensional scaffold, a medium recirculation loop comprising, preferably, at least one medium reservoir with additional in- and outlets.

Preferably, the bioreactor system comprises one or multiple solutions for on-line and/or in-line process monitoring and/or control including, but not limited to, in-line probes and/or sensors, mixing devices for fluids and/or gasses and feed and waste lines.

Preferably, the ratio between the specific surface area and the total volume of the fixed bed is comprised between 25 and 250, more preferably between 45 and 250, even more preferably between 50 and 250, advantageously between 60 and 250.

Preferably, the porous three-dimensional scaffold is different for the different steps of the method according to the present invention, for example a first porous three-dimensional scaffold is used during the seeding, attachment and expansion steps while a second porous three-dimensional scaffold is used during the production step. Indeed, different porous three-dimensional scaffolds may be used to optimize each step of the method according to the present invention.

Preferably, the total volume of the scaffold (or total fixed bed volume) comprises a first volume of scaffold material and a second volume of void inside the scaffold when the scaffold is not immerged in the culture medium and wherein a ratio between the second volume and the total fixed bed volume is comprised between 20 and 90%, more preferably between 25 and 85%.

Preferably, in the method according to the present invention, the ratio between the second volume of void inside the scaffold and a recirculation loop volume is comprised between 1% and 75%, more preferably between 20% and 50%, wherein the recirculation loop volume comprises a third volume and a fourth volume wherein the third volume is the volume of culture medium inside the bioreactor chamber (which includes the second volume) and the fourth volume is the volume of the culture medium in the associated recirculation loop between the outlet and the inlet of the bioreactor chamber. The volume of the optional reservoirs associated to the recirculation loop is not included in the fourth volume used for the calculation of the ratio between the second volume and the recirculation loop volume.

Alternatively, or in addition or preferably, the third volume of the culture medium inside the bioreactor chamber is smaller than the total volume of the scaffold (combining the first and second volume). Indeed, this permits to reduce the amount of nutrients to be added in the culture medium during the seeding, attachment, and/or expansion phase and accordingly the production costs.

Preferably, the ratio between the third volume of the culture medium inside the bioreactor and the total volume of the scaffold, is comprised between 20 and 99%, more preferably between 25 and 95%, more particularly, between 35 and 85%. Indeed, such ratios is useful to reduce the amount of culture medium used during the method of production while improving the yield of cell and extracellular vesicles (EVs) produced.

Advantageously, the method for the production of extracellular vesicles according to the present invention further comprises after the step of expanding the adherent cells and before the step of producing extracellular vesicles, replacing at least a portion of the culture medium in the bioreactor chamber with a culture medium of production, wherein the culture medium of production is a xeno-free medium or an animal-free medium. Indeed, a specific medium for EVs production which is xeno-free or an animal-free medium is optimal to obtain purified Evs of high quality.

Preferably, the method for the production of extracellular vesicles according to the present invention further comprises, after the harvesting of the extracellular vesicles, a step of purification of the extracellular vesicles. Indeed, a purification step may help obtaining extracellular vesicles with a high level of purity and of high quality.

Preferably, the ratio between the first volume of material of the scaffold and the third volume of the culture medium inside the bioreactor chamber is comprised between 10 and 85%, more preferably between 15 and 80%, more particularly, between 35 and 75%, and/or wherein the ratio between the first volume of material of the scaffold and the recirculation loop volume is comprised between 1% and 80%. Such volume ratios are convenient to promote a good balance between the amount of cell produced attached on the scaffold and the volume of culture medium which transport all the nutrients for cell growth.

Advantageously, the ratio between the second volume of void in the scaffold and the total volume of the scaffold is comprised between 20 and 90%, more preferably between 25 and 85%.

Preferably, in the method for the production of extracellular vesicles according to the present invention, the seeding step of the porous three-dimensional scaffold with a starting number of adherent cells resuspended after detachment is a dynamic seeding, wherein preferably the dynamic seeding comprises a step of seeding of the porous three-dimensional scaffold with adherent cells resuspended in a culture medium during a first predefined duration comprised between 0.5 h and 24 h, preferably around 2 h, at a flow rate with a vertical linear speed of perfusion comprised between 0.01 mm/s and 100 mm/s, preferably between 0.05 and 10 mm/s, the flow rate with a vertical linear speed of perfusion during the seeding step being preferably the same between the supply port and the outlet port and between the culture medium exit (outlet port) and the supply port ± 10%. Indeed, a dynamic seeding may improve the seeding efficiency of the scaffold by the cells of an adherent cell strain.

Preferably, in the method for the production of extracellular vesicles according to the present invention, the attachment of the adherent cells resuspended after detachment on the three-dimensional scaffold to form adherent cells on the porous three-dimensional scaffold is a dynamic attachment, wherein preferably the dynamic attachment comprises a step of attachment of adherent cells resuspended in a culture medium on the porous three-dimensional scaffold during a second predefined duration comprised between 0.5 h and 24 h, preferably around 2h, at a flow rate with a vertical linear speed of perfusion comprised between 0.01 mm/s and 100 mm/s, preferably between 0.05 and 10 mm/s, the flow rate with a vertical linear speed of perfusion during the attachment step being preferably the same between the supply port and the outlet port and between the culture medium exit (outlet port) and the supply port ± 10%. Indeed, a dynamic attachment may improve the attachment efficiency of the scaffold by the cells of an adherent cell strain.

Preferably, in the method for the production of extracellular vesicles according to the present invention, the expansion of the adherent cells on the porous three-dimensional up to a second number of adherent cells is an expansion in a fluidic dynamic environment, called here a dynamic expansion, wherein preferably the dynamic expansion comprises a step of expansion of the adherent cells on the porous three-dimensional scaffold during a growth phase during a third predefined duration comprised between 24h and 672h, preferably between 48h and 360h, more preferably between 72h and 300h, favourably around 240h, at a flow rate with a vertical linear speed of perfusion comprised between 0.01 mm/s and 100 mm/s, preferably between 0.05 and 10 mm/s.

Preferably, in the method for the production of extracellular vesicles according to the present invention, the production of extracellular vesicles by the adherent cells during a production phase, subsequent to or concomitantly with the growth phase upon stress and/or chemical induction is a dynamic production, wherein preferably the dynamic production comprises a step of production of extracellular vesicles during a fourth predefined duration comprised between 10 h and 672 h, preferably between 24h and 360h, favourably around 72h, at a flow rate with a vertical linear speed of perfusion comprised between 0.01 mm/s and 100 mm/s, preferably between 0.05 and 10 mm/s.

Preferably, the flow rate with a vertical linear speed of perfusion during the seeding step and/or during the attachment step and/or during the expansion step and/or during the production step is homogeneous throughout the bioreactor system, for example between the supply port and the outlet (port) and between the culture medium exit (outlet port) and the supply port.

Preferably, the method for the production of extracellular vesicles according to the present invention further comprises a continuous measurement of a series of culture parameters carried out between the outlet of medium (outlet port) and the supply of culture medium via the supply port, and a regulation by an analysis of the series of culture parameters to determine a deviation from a predefined standard and, in the presence of the deviation from the predefined standard, an adaptation of the culture parameters to reduce the deviation from the predefined standard. Indeed, a rapid adaptation of the series of culture parameters will improve the yield of cell growth and of EV production by ensuring in a constant manner an optimal environment depending on the steps of the method according to the present invention, i.e. seeding, attachment, expansion and/or production.

Preferably, in the method for the production of extracellular vesicles according to the present invention, the multiplication factor of the cells in a single expansion step is between 5 and 5000, preferably between 10 and 4000, more preferably between 15 and 1000.

Alternatively, or preferably, in the method for the production of extracellular vesicles according to the present invention, the multiplication factor during a single expansion step is at least 5, preferably at least 10, more preferably at least 15, even more preferably at least 40.

Preferably, in the method according to the present invention, during the attachment and/or the expansion of the adherent cells on the porous 3D scaffold and/or during the production of extracellular vesicles, oxygen is supplied to the culture medium at an amount sufficient for having a level of oxygen at the culture medium exit (outlet port) which is at least 50% of a level of oxygen in the supply port. Preferably, during the seeding step and/or during the attachment and/or during the expansion of the adherent cells on the porous 3D scaffold and/or during the production of extracellular vesicles, oxygen is supplied to the culture medium at an amount sufficient for having a level of oxygen at the outlet port which is equal or larger than the minimal oxygen level required for cell viability. Thus, cells will have a sufficient amount of oxygen anywhere in the 3D scaffold to ensure a proper growth and/or a proper production of extracellular vesicles.

Preferably, before the seeding of the porous three-dimensional scaffold with adherent cells resuspended after detachment, the porous three-dimensional scaffold is coated with one or more component chosen from the following group: glycoprotein, fibronectin, recombinant fibronectin, type I collagen, plant derived human recombinant collagen, proteoglycan, xeno-free attachment substrate, attachment substrate free of non-human animal derived components, or animal derived attachment substrate. Indeed, the treatment and/or the coating of the porous three-dimensional scaffold is used to improve the attachment of the cells on the scaffold.

Preferably, the supply port is located in a lower part of the bioreactor. Indeed, a supply port located in the lower part of the bioreactor is used to reduce the occurrence of preferred fluid pathways within the scaffold.

Preferably, the production phase having a fourth predefined duration is defined by a duration comprised between 24h and 672h, more preferably between 48h and 360h, favorably around 72h. Indeed, these durations are sufficiently long for an efficient production of extracellular vesicles while sufficiently short to limit cell mortality and/or to maintain the quality of the cell culture within the bioreactor.

The present invention also relates to a pharmaceutical composition containing extracellular vesicles obtained by the method according to the invention, under a liquid composition or a frozen composition or a lyophilized composition or a spray dried composition or a gel composition, wherein the extracellular vesicles are conditioned into a pharmaceutically acceptable liquid buffer.

According to the present invention, preferably, the term "an analysis of the culture conditions to determine a deviation from a predefined standard" is understood:
(1) a measurement of:
   (i) the amount of biological factors and/or one or more physico-chemical parameters of the culture medium such as temperature, pH, O2 level, CO2 level, metabolites, etc...,
   (ii) cell density on the porous three-dimensional scaffold and/or the glucose level and/or the lactate level within the bioreactor,
   (iii) the amount of extracellular vesicles present in the culture medium containing extracellular vesicles,
   (iv) cell viability,
   (v) the duration of cell culture and/or the duration of the extracellular vesicle production step,
(2) comparing this measurement to a corresponding predefined value and,
(3) determining a difference between this measurement and this corresponding predefined value.

According to the present invention, preferably, the term "an adaptation of the culture conditions to reduce the deviation from the predefined standard" is understood to mean: an adaptation of one or more physico-chemical parameters of the culture medium to reduce the difference between the measurement and the corresponding predefined value.

### Detailed description of the invention :

Other features and advantages of the present invention will be derived from the following non-limiting description, and with reference to the following figure:

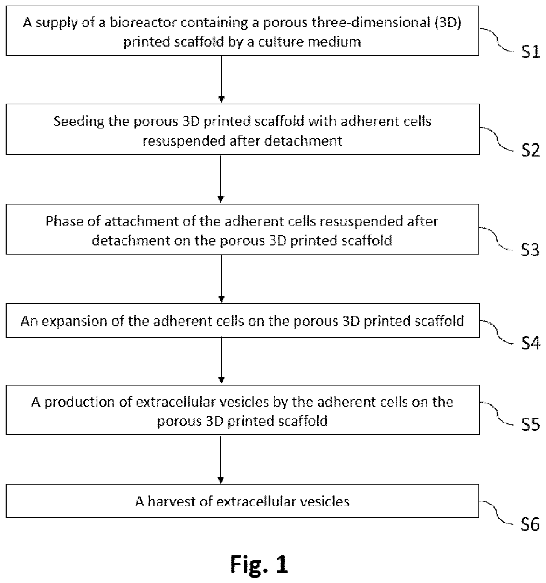

Figure 1 is a schematic representation of the different steps of the method for the production of extracellular vesicles according to the present invention.
Figure 2 shows a particle size distribution curve obtained in the production of extracellular vesicles according to the method of the present invention using immortalized Wharton's Jelly derived-mesenchymal stem cells (WJ-MSCs).

A first step S1 consists of a supply of a bioreactor chamber containing a porous three-dimensional scaffold by a culture medium via a supply port, and, after a predefined duration, a culture medium exit via an outlet port, wherein the porous three-dimensional scaffold having a specific surface area and a total volume. The total scaffold (fixed bed) volume comprising a first volume of fixed bed material and a second volume of void inside the fixed bed (when not immerged yet in the culture medium) and wherein the culture medium inside the bioreactor chamber having a third volume. Preferably, the supply port is located in a lower part of the bioreactor. The porous 3D scaffold is a porous 3D printed scaffold. In addition, a ratio surface/ volume between the specific surface area and the total fixed bed volume is comprised between 25 and 250, which is appropriate to give an adequate space to cells to growth on the specific surface area allowing to reach a large amount of cells with a high cell density, while maintaining a close contact between the adherent cells and the components, such as nutrients, growth factors, etc..., of the culture medium. In addition, the volume of the culture medium inside the bioreactor is limited contributing to produce extracellular vesicles at low cost, and also allowing to optimise the quantity of complementary molecules that can be added in the culture medium to promote cell growth and/or the production of extracellular vesicles. Moreover, since molecules secreted by cells play a role for the growth and/or for the secretion of their neighbouring cells, it is advantageous that these molecules reach a minimal concentration in the culture medium to have an impact on cell growth and/or on the production of extracellular vesicles. In that context, the fact that the ratio surface/volume is high and that the porous 3D scaffold is a 3D printed scaffold improve the cell growth conditions and the conditions of extracellular vesicles production by these cells by increasing the concentration of secreted molecules by the cells in culture and by allowing a uniform cell culture within the 3D scaffold. Preferably, the predefined duration is comprised between 0.5 h and 672h.

In a preferred embodiment, the supply of the bioreactor chamber containing a porous three-dimensional printed scaffold is performed with a culture medium fed via a supply port, preferably located in the bottom of the bioreactor chamber and exiting the bioreactor chamber through an outlet port, preferably located at the top of the bioreactor chamber thereby forming a perfused (dynamic) bioreactor by circulation of culture medium from the supply port to the outlet port and from the outlet port to the supply port.

Preferably, the supply of the bioreactor chamber containing a porous three-dimensional printed scaffold with a culture medium fed via a supply port, preferably located in the bottom of the bioreactor chamber, and the exit of the culture medium from the bioreactor chamber through an outlet port, preferably located at the top of the bioreactor chamber, is a synchronous, continuous, process forming a perfused bioreactor.

Alternatively, the supply of the bioreactor chamber containing a porous three-dimensional printed scaffold with a culture medium fed via a supply port, preferably located in the bottom of the bioreactor chamber, and the exit of the culture medium from the bioreactor chamber through an outlet port, preferably located at the top of the bioreactor chamber, is an asynchronous process, for example a first supply of the bioreactor chamber with a culture medium fed via a supply port, preferably located in the bottom of the bioreactor chamber, happens first followed after a predefined duration by the exit of the culture medium from the bioreactor chamber through an outlet port, preferably located at the top of the bioreactor chamber, defining a duration of static cell culture during the predefined duration.

Preferably, the first volume of scaffold material and the second volume of void inside the scaffold is defined by a ratio second volume: total volume comprised between 20 % and 90%. Indeed, a high ratio second volume: total volume defines a small volume of void inside the scaffold as compared to the volume of the material of the scaffold, which may be favourable, for example during a phase of extracellular vesicles production by adherent cells on the scaffold. However, a low ratio second volume: total volume defines a large volume of void inside the scaffold as compared to the volume of the material of the scaffold, which may be favourable during a phase a cell growth and/or cell expansion.

Preferably, the ratio between the second volume of void inside the scaffold and a recirculation loop volume is comprised between 1% and 75%, more preferably between 20% and 50 wherein the recirculation loop volume comprises a third volume and a fourth volume wherein the third volume is the volume of culture medium inside the bioreactor chamber (which includes the second volume) and the fourth volume is the volume of the culture medium in inlet and outlet tubing of the bioreactor chamber, being the associated recirculation loop and excluding the volume of culture medium in an optional recirculation reservoir.

Advantageously, the supply of a bioreactor chamber containing a porous three-dimensional scaffold by a culture medium via a supply port is performed with a flow rate at a linear speed comprised between 0.01 and 100 mm/s, preferably between 0.05 and 10 mm/s. Advantageously, the culture medium exit is performed at a same linear speed than the linear speed of the supply of the bioreactor chamber by the culture medium via the supply port in order to form a perfused bioreactor by circulation of culture medium from said culture medium supply to said culture medium outlet and from said culture medium outlet to said culture medium supply. Advantageously, the supply and/or the outlet of the culture medium is carried out at a flow rate with a linear speed between 0.01 and 100 mm/s, preferably between 0.05 and 10 mm/s. Advantageously, a series of pumps is connected to the bioreactor to ensure the supply of the bioreactor chamber by a culture medium via the supply port and/or to ensure the culture medium exit with an appropriate flow rate. Preferably, the porous three-dimensional scaffold is a three-dimensional printed scaffold using a biocompatible polymer, for example a UV curable resin. Preferably, before the seeding of the porous three-dimensional scaffold with adherent cells resuspended after detachment, the porous three-dimensional scaffold is treated, for example with a plasma or UV treatment, and/or the porous three-dimensional scaffold is coated with one or more proteins chosen from the following group: glycoprotein, fibronectin, recombinant fibronectin, type I collagen, proteoglycan, plant derived human recombinant collagen, xeno-free attachment substrate or attachment substrate free of non-human animal derived components.

In step S2, the seeding of the porous three-dimensional scaffold with a starting number of adherent cells resuspended after detachment by feeding the starting number of adherent cells resuspended after detachment in a volume of culture medium. Preferably, the method for the production of extracellular vesicles according to the present invention is characterized by a seeding efficiency between 50 and 95%, wherein the seeding efficiency corresponds to the number of cells seeded and attached on the porous 3D scaffold as compared to the number of adherent cells resuspended after detachment present in the volume of culture medium supplied in the bioreactor chamber.

Preferably, the seeding of the porous three-dimensional scaffold with adherent cells resuspended after detachment is performed by feeding the adherent cells resuspended after detachment in a volume of culture medium with a cell density comprised between 100 cells/cm² and 30000 cells/cm² (number of cells : specific surface area of the porous 3D scaffold based on the CAD design), more preferably between 500 cells/cm² and 20000 cells/cm², or alternatively comprised between 5000 cells/cm³ and 4000000 cells/cm³ (number of cells : total volume of porous 3D scaffold or total fixed bed volume). Preferably, the adherent cells are tumoral or non-tumoral immortalized cells forming part of a group of cells comprising immortalized immune regulatory cells, immortalized mesenchymal stem cells, immortalized Wharton's Jelly derived-mesenchymal stem cells, immortalized bone marrow-derived mesenchymal stromal cells, immortalized hTERT-mesenchymal stem cells, and/or immortalized induced pluripotent stem cells or cells derived from induced pluripotent stem cells. Preferably, the seeding of the porous three-dimensional scaffold with adherent cells resuspended after detachment is a dynamic seeding, wherein the dynamic seeding consists of seeding the porous 3D scaffold with adherent cells resuspended after detachment in a constant perfused bioreactor. More preferably the dynamic seeding comprises a step of seeding of the porous three-dimensional scaffold with adherent cells resuspended in a culture medium during a first predefined duration comprised between 0.5 h and 24 h, preferably around 2h, at a flow rate with a linear speed comprised between 0.01 and 100 mm/s.

In step S3, preferably, after the first predefined duration, the culture medium is replaced by a fresh culture medium and the bioreactor is perfused with the fresh culture medium at a flow rate with a linear speed comprised between 0.01 and 100 mm/s, preferably between 0.05 and 10 mm/s during a second predefined duration corresponding to the phase of attachment of the adherent cells resuspended after detachment. Preferably, the attachment of the adherent cells resuspended after detachment on the three-dimensional scaffold to form adherent cells on the porous three-dimensional scaffold is a dynamic attachment, wherein preferably said dynamics attachment comprises a step of attachment of adherent cells resuspended in a culture medium on the porous three-dimensional scaffold during a second predefined duration comprised between 0.5 h and 24 h, preferably around 2h, at a flow rate with a linear speed comprised between 0.01 and 100 mm/s, preferably between 0.05 and 10 mm/s.

Step S4, is the expansion of the adherent cells on the porous three-dimensional scaffold up to a second number of adherent cells, the second number of adherent cells divided by said starting number defining a multiplication factor, wherein the multiplication factor is at least 5 during a single expansion step in said scaffold. Preferably, the expansion is a dynamic expansion, wherein preferably the dynamic expansion comprises a step of expansion of the adherent cells on the porous three-dimensional scaffold during a growth phase during a third predefined duration comprised between 24h and 672 h, preferably between 48h and 360h, more preferably between 72h and 300h, favourably around 240h, at a flow rate with a linear speed comprised between 0.01 and 100 mm/s, preferably between 0.05 and 10 mm/s, the linear speed during the expansion step being preferably the same between the supply port and the culture medium exit (outlet port) and between the culture medium exit and the supply port ± 10%.

Alternatively, the seeding of the porous three-dimensional scaffold with adherent cells resuspended after detachment is a static seeding wherein the static seeding corresponds to a seeding phase where the bioreactor is not perfused. Preferably, the seeding efficiency corresponding to the number of cells seeded on the porous 3D scaffold divided by the number of cells, and/or the phase of attachment of the adherent cells resuspended after detachment on the porous 3D scaffold is a static attachment wherein the static attachment corresponds to an attachment phase where the bioreactor is not perfused, and/or the expansion of the adherent cells on the porous 3D scaffold is a static expansion wherein the static expansion corresponds to an expansion phase where the bioreactor is not perfused.

Preferably, in step S4 corresponding to the expansion of the adherent cells on the porous 3D scaffold, the multiplication factor is at least 5 during a single expansion step in said scaffold, preferably at least 15, more preferably at least 40.

Preferably, the seeding of the porous three-dimensional (3D) scaffold with adherent cells resuspended after detachment (step S2), the phase of attachment of the adherent cells resuspended after detachment on said three-dimensional scaffold (step S3) and the expansion of the adherent cells on the porous three-dimensional scaffold during a growth phase (step S4) are performed with a culture medium adapted for the growth of mesenchymal stem cells in the presence or absence of serum and in the presence or absence of culture medium complements, such as for example the commercial media formulations provided by RoosterBio, Takara, Stemcell, Gibco, Lonza, ....

Step S5 consists of a production of extracellular vesicles by the adherent cells during a production phase, subsequent to or concomitantly with the growth phase during which the vesicles are produced and excreted in the culture medium to give a culture medium enriched with extracellular vesicles. Preferably, the production phase is subsequent to the growth phase, wherein the production phase is initiated upon stress and/or chemical induction, during which the vesicles are produced and excreted in the culture medium to give a culture medium enriched with extracellular vesicles. Alternatively, the production phase is concomitant to the growth phase in the presence or in the absence of stress and/or chemical induction, during which the vesicles are produced and excreted in the culture medium to give a culture medium enriched with extracellular vesicles.

Preferably, after the step S4 of expanding the adherent cells and before the step S5 of producing extracellular vesicles, a replacement of at least a portion of the culture medium in the bioreactor with a culture medium of production is performed, wherein the culture medium of production is a xeno-free medium. Preferably, the culture medium is different from the production culture medium. Preferably, the production culture medium for the production of extracellular vesicles is a xeno-free culture medium or an animal-free medium. Preferably, the production phase is defined by a duration comprised between 24h and 672h, more preferably between 48h and 360h, favorably around 72h. Preferably, the production of extracellular vesicles by the adherent cells during a production phase, subsequent to the growth phase upon stress and/or chemical induction is a dynamic production, wherein preferably the dynamic production comprises a step of production of extracellular vesicles during a fourth predefined duration comprised between 10 h and 672 h, preferably between 24h and 360h, favourably around 72h, at a flow rate with a linear speed comprised between 0.01 and 100 mm/s, preferably between 0.05 and 10 mm/s, the linear speed during the production step being preferably the same between the supply port and the outlet port and between the outlet port and the supply port ± 10%. Preferably, during the step S4 of expansion of the adherent cells on the porous 3D scaffold and the step S5 of production of extracellular vesicles by the adherent cells during a production phase, if an oxygen gradient (O₂ gradient) is present within the bioreactor between the supply of the culture medium via the supply port and the culture medium outlet, the level of oxygen at the culture medium outlet is at least 50% of the level of oxygen in the supply port. Preferably, during the seeding step (step S2) and/or the attachment (step S3) and/or the expansion of the adherent cells on the porous 3D scaffold (step S4) and/or during the production of extracellular vesicles (step S5), oxygen is supplied to the culture medium at an amount sufficient for having a level of oxygen at the culture medium outlet which is equal or larger than the minimal oxygen level required for cell viability. Preferably, the number of extracellular vesicles produced per adherent cell on the porous 3D scaffold during the production phase of the method for the production of extracellular vesicles according to the present invention is comprised between 1E3 and 1E7 extracellular vesicles/cell. Preferably, the number of extracellular vesicles produced per cm² of specific surface of the porous 3D scaffold during the production phase of the method according to the present invention is between 1E9 and 1E12 extracellular vesicles/cm². Preferably, a number of cell / cm³ of the total volume of the scaffold is comprised between 250000 (2.5E5) cells/cm³ and 100000000 (1E8) cells/cm³ during the production phase of the method for the production of extracellular vesicles according to the present invention. Preferably, a number of cell / cm² of the specific surface of the scaffold is comprised between 25000 (2.5E4) cells/cm² and 500000 (5E5) cells/cm² during the production phase of the method for the production of extracellular vesicles according to the present invention.

Step S6 consists of a harvest of extracellular vesicles. Preferably, after the harvesting of the extracellular vesicles, a step of purification of the extracellular vesicles is performed.

Preferably, the different steps S1 to S5 of the method for the production of extracellular vesicles according to the present invention, or at least step S1, or at least step S2, or at least step S3, or at least step S4, or at least step S5, or at least step S6 further comprises a continuous measurement of a series of culture parameters, preferably carried out between the outlet of medium and the supply of culture medium by a series of sensors, and a regulation by an analysis of the series of culture parameters to determine a deviation from a predefined standard and, in the presence of the deviation from the predefined standard, an adaptation of the culture parameters to reduce the deviation from the predefined standard. Alternatively, the continuous measurement of a series of culture parameters is carried out directly by a series of sensors located within the bioreactor.

The present invention also relates to a pharmaceutical composition containing extracellular vesicles obtained by the method according to the invention, under a liquid composition or a frozen composition or a lyophilized composition or a spray dried composition or a gel composition, wherein the extracellular vesicles are conditioned into a pharmaceutically acceptable liquid buffer.

### Examples.-

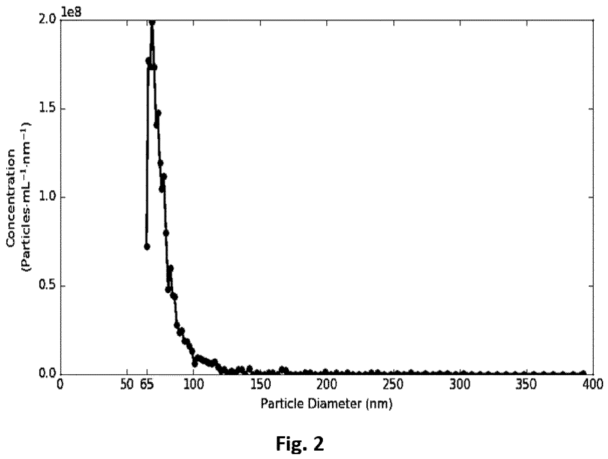

The examples below describe the method for the production of extracellular vesicles compatible with the pharmaceutical industry according to the present invention using immortalized Bone Marrow (BM-MSCs) and Wharton's Jelly derived-mesenchymal stem cells (WJ-MSCs) in a perfusion bioreactor system using a 3D printed porous scaffold (fixed bed) with a ratio of 0.85 between the first volume of scaffold material and the second volume of void inside the scaffold. The material of the scaffold is a UV curable resin. The specific surface area of the 3D printed porous scaffold is about 48 cm²/cm³ (specific surface area of the scaffold: total volume of the scaffold). Within these examples the bioreactor further comprises a tubing set connecting the supply port of the bioreactor to a medium reservoir. Upstream from the bioreactor a dedicated pump-tubing is used for mounting it on a peristaltic pump while downstream from the bioreactor a gas permeable tube is used to enable the exchange of oxygen and CO₂ in the controlled environment of a cell culture incubator. The ratio of the second volume of void inside the scaffold and a total volume is approximately 0.05. The conditions given in the Examples 1 to 17 here below gave yields of extracellular vesicles produced per cm² comprised between 2E+09 and 2.9E+10. Examples of the particles size distribution obtained are presented on Figure 2.

**Example 1.-** Production of extracellular vesicles according to the method of the present invention using immortalized Wharton's Jelly derived-mesenchymal stem cells (WJ-MSCs) in a perfusion bioreactor using a 3D printed scaffold (fixed bed).

A protein coating step is performed on the 3D scaffold to enable and improve cell attachment to the 3D printed surface, specific surface area). In this example, the scaffold (fixed bed) is coated with a commercial available human recombinant collagen by impregnated the scaffold with the coating solution for 2h at room temperature after which the excess was rinsed away using DPBS.

A seeding suspension containing 1.4E5 cells/ml (immortalized Wharton's Jelly derived-mesenchymal stem cells, WJ-MSCs) was prepared in a commercial MSC xeno-free cell culture medium. A total of 200 µl of seeding suspension per cm² of scaffold (fixed bed) was recirculated for 2 hrs at a flow rate with a vertical linear speed of 0.277 mm/s.

After seeding, the cell suspension was replaced by fresh cell culture medium and the flow rate was reduced to a flow rate with a linear speed of 0.111 mm/s. For a duration of 6 days the seeded WJ-MSCs were subsequently expanded under an atmosphere with ambient oxygen concentrations, 5% CO₂ at 37°C and medium was refreshed every 2-3 days.

After the growing phase, the culture medium was exchanged for extracellular vesicles (EV) induction medium. For the Example1, a commercial medium optimized for extracellular vesicles production was used. Perfusion was continued at a flow rate with a linear speed identical as during the expansion phase. After 1 day an additional refresh of the EV medium was performed and the EV production phase was initiated for 3 days. The resulting medium was collected for EV quantification for which it was spun down at 2000g for 10 minutes after which the supernatant was stored at -80°C prior to analysis.

EV quantification has been performed using Microfluidic Resistive Pulse Sensing (MRPS) showed final concentrations up to 2.9E10 particles/cm² have been reached during the 3 day induction phase.

**Example 2.-** Production of extracellular vesicles according to the method of the present invention using immortalized Wharton's Jelly derived-mesenchymal stem cells (WJ-MSCs) in a perfusion bioreactor using a 3D printed scaffold (fixed bed) using a DMEM based medium formulation for the induction of the EV production.

Example 1 has been reproduced except that a DMEM based medium formulation (without Fetal Bovine Serum) has been used for the EV induction (step of production of EV).

**Example 3.-** Production of extracellular vesicles according to the method of the present invention using immortalized Wharton's Jelly derived-mesenchymal stem cells (WJ-MSCs) in a perfusion bioreactor using a 3D printed scaffold.

Example 1 has been reproduced except that during the EV production phase a 50% medium refresh was done daily.

**Example 4.-** Production of extracellular vesicles according to the method of the present invention using immortalized Wharton's Jelly derived-mesenchymal stem cells (WJ-MSCs) in a perfusion bioreactor using a 3D printed scaffold using a DMEM based medium formulation for the induction of the EV production.

Example 2 has been reproduced except that during the EV production phase a 50% medium refresh was done daily.

**Example 5.-** Production of extracellular vesicles according to the method of the present invention using immortalized Wharton's Jelly derived-mesenchymal stem cells (WJ-MSCs) in a perfusion bioreactor using a 3D printed scaffold using a prolonged, 6-day induction phase (step of production of extracellular vesicles).

Example 1 has been reproduced except that the fourth predefined duration (total duration of the EV production phase) was prolonged to 6 days. Over the course of these 6 days no refresh of the EV induction medium was performed.

**Example 6.-** Production of extracellular vesicles according to the method of the present invention using immortalized Wharton's Jelly derived-mesenchymal stem cells (WJ-MSCs) in a perfusion bioreactor using a 3D printed scaffold using a DMEM based medium formulation using a prolonged, 6-day induction phase (fourth predefined duration).

Example 2 has been reproduced except that the total duration of the EV production phase was prolonged to 6 days. Over the course of these 6 days no refresh of the EV induction medium was performed.

**Example 7.-** Production of extracellular vesicles according to the method of the present invention using immortalized Wharton's Jelly derived-mesenchymal stem cells (WJ-MSCs) in a perfusion bioreactor using a 3D printed scaffold using a prolonged, 6-day induction phase.

Example 5 was reproduced except that, at 3 days of EV induction, a medium refresh was performed to sustain the EV production further during the 2^{nd} set of 3 days of EV induction.

**Example 8.-** Production of extracellular vesicles according to the method of the present invention using immortalized Wharton's Jelly derived-mesenchymal stem cells (WJ-MSCs) in a perfusion bioreactor using a 3D printed scaffold using a DMEM based medium formulation using a prolonged, 6-day induction phase.

Example 6 was reproduced except that at 3 days of EV induction a medium refresh was performed to sustain the EV production further during the 2^{nd} set of 3 days of EV induction.

**Example 9.-** Production of extracellular vesicles according to the method of the present invention using immortalized Wharton's Jelly derived-mesenchymal stem cells (WJ-MSCs) in a perfusion bioreactor using a 3D printed scaffold using a FBS supplemented medium formulation for the cell expansion phase.

Example 1 was reproduced except that the culture medium used was a commercial MSC xeno-free cell culture medium supplemented with Fetal Bovine Serum.

**Example 10.-** Production of extracellular vesicles according to the method of the present invention using immortalized Wharton's Jelly derived-mesenchymal stem cells (WJ-MSCs) in a perfusion bioreactor using a 3D printed scaffold using an FBS supplemented medium formulation for the cell expansion phase and a DMEM based medium formulation for the induction of the EV production (step of production of EV).

Example 9 was reproduced except that the culture medium used was a DMEM based medium formulation (without Fetal Bovine Serum addition) for the EV induction.

**Example 11.-** Production of extracellular vesicles according to the method of the present invention using immortalized Wharton's Jelly derived-mesenchymal stem cells (WJ-MSCs) in a perfusion bioreactor using a 3D printed scaffold using an FBS supplemented medium formulation for the cell expansion phase and a DMEM based medium formulation for the induction of the EV production.

Example 2 has been reproduced except that the commercial MSC xeno-free cell culture medium used during the expansion was supplemented with foetal bovine serum.

**Example 12.-** Production of extracellular vesicles according to the method of the present invention using immortalized Bone Marrow derived-mesenchymal stem cells (BM-MSCs) in a perfusion bioreactor using a 3D printed scaffold, an FBS supplemented medium formulation for the cell expansion phase and a DMEM based medium formulation for the induction of the EV production

Example 1 has been reproduced except that an immortalized Bone Marrow derived-mesenchymal stem cell source was used in combination with an FBS supplemented medium formulation for the expansion phase and a DMEM based medium formulation for the induction of the EV production.

**Example 13.-** Production of extracellular vesicles according to the method of the present invention using immortalized Bone Marrow derived-mesenchymal stem cells (BM-MSCs) in a perfusion bioreactor using a 3D printed scaffold, an FBS supplemented medium formulation for the cell expansion phase and a commercial medium optimized for extracellular vesicles production for the induction of the EV production.

Example 12 was reproduced except that a commercial xeno-free medium optimized for extracellular vesicles production was used for the EV production.

**Example 14.-** Production of extracellular vesicles according to the method of the present invention using immortalized Bone Marrow derived-mesenchymal stem cells (BM-MSCs) in a perfusion bioreactor using a 3D printed scaffold, a commercial xeno-free culture medium formulation for the cell expansion phase and a commercial medium optimized for extracellular vesicles production for the induction of the EV production.

Example 13 was reproduced except that a commercial medium xeno-free for MSC expansion was used during the expansion phase of the cells.

**Example 15.-** Production of extracellular vesicles according to the method of the present invention using immortalized Bone Marrow derived-mesenchymal stem cells (BM-MSCs) in a perfusion bioreactor using a 3D printed scaffold, a commercial xeno-free culture medium formulation for the cell expansion phase and a DMEM based medium formulation for the induction of the EV production.

Example 14 was reproduced except that a DMEM based medium formulation was used for the EV production.

**Example 16.-** Production of extracellular vesicles according to the method of the present invention using immortalized Bone Marrow derived-mesenchymal stem cells (BM-MSCs) in a perfusion bioreactor using a 3D printed scaffold, a commercial xeno-free culture medium formulation for the cell expansion phase and a DMEM based medium formulation for the induction of the EV production.

Example 15 was reproduced except that a commercial medium xeno-free for MSC expansion, other than was used in example 15, was used during the initial expansion phase of the cells.

**Example 17.-** Production of extracellular vesicles according to the method of the present invention using immortalized Bone Marrow derived-mesenchymal stem cells (BM-MSCs) in a perfusion bioreactor using a 3D printed scaffold, a commercial xeno-free culture medium formulation for the cell expansion phase and a DMEM based medium formulation for the induction of the EV production.

Example 16 was reproduced except that a DMEM based medium formulation was used for the EV production. For conditions described in examples 12 to 17 cell expansion folds of at least 10X were obtained after the EV production phase in the perfusion bioreactor setup. In comparison, static expansion (in absence of medium perfusion) of the BM-MSCs for an equal period yielded 3 to 5 times lower final cell densities.

**Example 18.-** Expansion of an immortalized Bone Marrow derived-mesenchymal stem cells (BM-MSCs) in a perfusion bioreactor using a 3D printed scaffold 10X the size as was used in prior examples, using an FBS supplemented medium formulation for a period of 14 days.

Example 12 was repeated with the exclusion of the EV production phase. Additionally, a fixed bed 10X the size of what was used in the prior examples was used and volumes and concentrations were scaled accordingly. Based on metabolic measurements at least a 16X multiplication of the expanding cell population was observed.

It is understood that the present invention is in no way limited to the embodiments described above and that many modifications can be made thereto without departing from the scope of the appended claims.

## Claims

1. Method for the production of extracellular vesicles compatible with the pharmaceutical industry, for example exosomes comprising the following steps:
- a supply of a bioreactor chamber containing a porous three-dimensional scaffold by a cell culture medium via a supply port, and, after a predefined duration, an exit via an outlet port, wherein the porous three-dimensional scaffold having a specific surface area and a total volume,
- seeding said porous three-dimensional scaffold with a starting number of adherent cells resuspended after detachment by feeding said starting number of adherent cells resuspended after detachment in a volume of culture medium,
- a phase of attachment of said adherent cells resuspended after detachment on said three-dimensional scaffold to form adherent cells on said porous three-dimensional scaffold,
- an expansion of the adherent cells on said porous three-dimensional scaffold up to a second number of adherent cells, said second number of adherent cells divided by said starting number defining a multiplication factor,
- a production of extracellular vesicles by said adherent cells during a production phase, subsequent to or concomitantly with the expansion, during which the vesicles are produced and excreted in the culture medium to give a culture medium enriched with extracellular vesicles,
- a harvest of extracellular vesicles,
**characterized in that**
said porous three-dimensional scaffold is a porous three-dimensional printed scaffold having a ratio between said specific surface area and said total volume of the scaffold comprised between 25 and 250, on which said adherent cells expanded are immortalized cells from an immortalized cell line, said immortalized cells having the multiplication factor of at least 5 for a single expansion step in the said scaffold.

2. Method according to claim 1, wherein said adherent cells are tumoral or non-tumoral immortalized cells, preferably tumoral or non-tumoral immortalized cells forming part of a group of cells comprising immortalized immune regulatory cells, immortalized mesenchymal stem cells, immortalized Wharton's Jelly derived-mesenchymal stem cells, immortalized bone marrow-derived mesenchymal stromal cells, immortalized hTERT-mesenchymal stem cells, and/or immortalized induced pluripotent stem cells or cells derived from induced pluripotent stem cells.

3. Method according to claim 1 or claim 2, wherein said total volume comprises a first volume of scaffold material and a second volume of void inside the scaffold when the scaffold is not immerged in said culture medium and wherein a ratio between said second volume and said total volume is comprised between 20 and 90%, more preferably between 25 and 85%.

4. Method according to anyone of the preceding claims, wherein said multiplication factor is at least 5 during a single expansion step in said scaffold, preferably at least 10, more preferably at least 15, even more preferably at least 40.

5. Method according to anyone of the preceding claims, wherein the ratio between said second volume of void inside the scaffold and a recirculation loop volume is comprised between 1% and 75%, more preferably between 20% and 50%, wherein said recirculation loop volume comprises a third volume and a fourth volume wherein said third volume is the volume of culture medium inside said bioreactor chamber, which comprises said second volume, and said fourth volume is the volume of the culture medium in the associated recirculation loop between the outlet and the inlet of the bioreactor chamber.

6. Method according to anyone of the preceding claims, further comprising, after the step of expanding the adherent cells and before the step of producing extracellular vesicles, replacing at least a portion of said culture medium in said bioreactor chamber with a culture medium of production, wherein the culture medium of production is a xeno-free medium or an animal-free medium.

7. Method according to anyone of the preceding claims, further comprising, after said harvesting of the extracellular vesicles, a step of purification of the extracellular vesicles.

8. Method according to any one of the preceding claims, in which (i) said seeding of the porous three-dimensional scaffold by means of cells of an adherent cell strain is a dynamic seeding, wherein preferably the dynamic seeding comprises a step of seeding of the porous three-dimensional scaffold with adherent cells resuspended in a culture medium during a first predefined duration comprised between 0.5 h and 24 h, preferably around 2h, at a flow rate with a linear speed of perfusion comprised between 0.01 mm/s and 100 mm/s, preferably between 0.05 and 10 mm/s, and/or (ii) said attachment of said adherent cells resuspended after detachment on said three-dimensional scaffold to form adherent cells on said porous three-dimensional scaffold is a dynamic attachment, wherein preferably said dynamics attachment comprises a step of attachment of adherent cells resuspended in a culture medium on the porous three-dimensional scaffold during a second predefined duration comprised between 0.5 h and 24 h, preferably around 2h, at a flow rate with a linear speed of perfusion comprised between 0.01 mm/s and 100 mm/s, preferably between 0.05 and 10 mm/s, and/or (iii) said expansion of the adherent cells on said porous three-dimensional scaffold during a growth phase is a dynamic expansion, wherein preferably the dynamic expansion comprises a step of expansion of the adherent cells on said porous three-dimensional scaffold during a growth phase during a third predefined duration comprised between 24h and 672 h, preferably between 48h and 360h, more preferably between 72h and 300h, favourably around 240h, at a flow rate with a linear speed of perfusion comprised between 0.01 mm/s and 100 mm/s, preferably between 0.05 and 10 mm/s, and/or (iv) said production of extracellular vesicles by said adherent cells during a production phase, subsequent to the growth phase upon stress and/or chemical induction is dynamic production, wherein preferably the dynamic production comprises a step of production of extracellular vesicles during a fourth predefined duration comprised between 10 h and 672 h, preferably between 24h and 150h, favourably around 72h, at a flow rate with a linear speed of perfusion comprised between 0.01 mm/s and 100 mm/s, preferably between 0.05 and 10 mm/s.

9. Method according to anyone of the preceding claims, further comprising a continuous measurement of a series of culture parameters carried out between the outlet of medium and the alimentation of culture medium, and a regulation by an analysis of said series of culture parameters to determine a deviation from a predefined standard and, in the presence of said deviation from said predefined standard, an adaptation of the culture parameters to reduce said deviation from said predefined standard.

10. Method according to any one of the preceding claims, wherein during said attachment and/or said expansion of the adherent cells on the porous 3D scaffold and/or during said production of extracellular vesicles, oxygen is supplied to the culture medium at an amount sufficient for having a level of oxygen at the culture medium outlet which is at least 50% of a level of oxygen in the supply port, or during said seeding step and/or during said attachment and/or during said expansion of the adherent cells on the porous 3D scaffold and/or during said production of extracellular vesicles, oxygen is supplied to the culture medium at an amount sufficient for having a level of oxygen at the culture medium outlet which is equal or larger than the minimal oxygen level required for cell viability.

11. Method according to anyone of the preceding claims, wherein the ratio between said first volume of material of the scaffold and said third volume of the culture medium inside the bioreactor chamber is comprised between 10 and 85%, more preferably between 15 and 80%, more particularly, between 35 and 75%, and/or wherein the ratio between the first volume of material of the scaffold and the recirculation loop volume is comprised between 1% and 80%.

12. Method according to any one of the preceding claims, in which before the seeding of the porous three-dimensional scaffold with adherent cells resuspended after detachment, the porous three-dimensional scaffold is coated with one or more proteins chosen from the following group: glycoprotein, fibronectin, recombinant fibronectin, type I collagen, plant derived human recombinant collagen, proteoglycan, xeno-free attachment substrate or attachment substrate free of non-human animal derived components.

13. Method according to any one of the preceding claims, in which said supply port is located in a lower part of said bioreactor.

14. Method according to any one of the preceding claims, wherein said production phase having a fourth predefined duration is defined by a duration comprised between 24h and 672h, more preferably between 48h and 360h, favorably around 72h.

15. Pharmaceutical composition containing extracellular vesicles obtained by the method according to any one of claims 1 to 14, under a liquid composition or a frozen composition or a lyophilized composition or a spray dried composition or a gel composition, wherein the extracellular vesicles are conditioned into a pharmaceutically acceptable liquid buffer.
